Europäisches Patentamt

European Patent Office

Office européen des brevets

(19)

(11) Veröffentlichungsnummer : **0 061 108**
**B1**

(12) # EUROPÄISCHE PATENTSCHRIFT

(45) Veröffentlichungstag der Patentschrift :
**30.12.86**

(21) Anmeldenummer : **82102044.3**

(22) Anmeldetag : **13.03.82**

(51) Int. Cl.⁴ : **A 61 F  2/00**, A 61 K  9/20,
A 61 L 15/06

(54) **Knochenimplantat aus Tricalciumphosphat mit mikro-und makroporöser Struktur, Verfahren zu seiner Herstellung und seiner Verwendung.**

(30) Priorität : **19.03.81 DE 3110681**
**02.09.81 DE 3134728**

(43) Veröffentlichungstag der Anmeldung :
**29.09.82 Patentblatt 82/39**

(45) Bekanntmachung des Hinweises auf die Patenterteilung : **30.12.86 Patentblatt 86/52**

(84) Benannte Vertragsstaaten :
**AT BE CH FR GB IT LI NL SE**

(56) Entgegenhaltungen :
**EP-A- 0 003 979**
**EP-A- 0 013 077**
**DE-B- 2 511 122**
**PHILIPS TECHNISCHE RUNDSCHAU, Band 37, Nr. 9/10, 1977/78 Eindhoven J.G.J. PEELEN et al. "Gesinterter Hydroxylapatit als Biokeramik" Seiten 255 bis 257**
**Clinical Orthopaedics and Related Research, Nr. 143, 1979, S. 266 bis 270**

(73) Patentinhaber : **mundipharma GmbH**
**Mundipharma Strasse 2**
**D-6250 Limburg (Lahn) 1 (DE)**

(72) Erfinder : **Eitenmüller, Jürgen, Dr. med.**
**Römerstrasse 27**
**D-5024 Brauweiler (DE)**

(74) Vertreter : **Eisenführ & Speiser**
**Martinistrasse 24**
**D-2800 Bremen 1 (DE)**

EP 0 061 108 B1

## Beschreibung

Die Erfindung betrifft ein Knochenimplantat aus Tricalciumphosphatkeramik mit mikroporöser und makroporöser Struktur, welches mit mindestens einem Breitbandmikrobizid imprägniert ist. Die Erfindung betrifft weiterhin ein Verfahren zum Herstellen eines solchen Knochenimplantats.

Als Material für Knochenimplantate haben sich insbesondere die völlig inerten Metalle, hochgeglühte Aluminium-oxide, aber auch Kunststoffe wie Polymethylmethacrylat und Polymethylmethacrylat-Methylacrylatcopolymere bewährt. Insbesondere diese organischen Polymere lassen sich erfolgreich mit Breitbandmikrobiziden imprägnieren und haben sich in der Praxis durchaus bewährt ; vgl. Wahlig und Buchholz, Chirurg 43, S. 441-445 (1972).

Besondere Beachtung haben in der letzten Zeit auch gesinterte Hydroxylappatite bzw. Tricalciumphosphate erlangt, die je nach dem Grad ihrer Makroporosität und Mikroporosität mehr oder weniger gut mit der Knochensubstanz verwachsen bzw. sogar vom Knochen resorbiert werden ; vgl. Peelen, Reijda und de Groot, Ceramurgia International, Vol. 4, N. 2, 1978, Seite 71 sowie de Groot, Biomaterials 1980, Vol. 1, Seite 47. Mit zunehmender Porosität wächst auch die Abbaubarkeit dieses Materials, jedoch sinkt in gleichem Maße die mechanische Stabilität.

Es ist auch schon vorgeschlagen worden, aus einem Matrix-material aus resorbierbaren gesinterten Calciumphosphaten, die aus Calciumoxid und $P_2O_5$ in einem Mengenverhältnis von 5 : 1 und 2 : 1 zusammengesetzt sind und einem Antibiotikum, ein Depot gesteuerter Pharmakaabgabekinetik herzustellen. Für die praktische Applikation wurde weiter vorgeschlagen, die so erhaltenen Pulver mit Hilfsstoffen zu überziehen oder mikrozuverkapseln. Diese Materialien haben aber bisher keinen Eingang in die Praxis gefunden.

Die EP-A-0 003 979 beschreibt ein implantierbares Pharmaka-Depot und ein Verfahren zu dessen Herstellung. Grundlage für dieses bekannte Implantat ist eine Matrix aus Calciumphosphat, das mit einem Wirkstoff imprägniert ist und einen Hilfsstoff zur Erzeugung der Depot-Wirkung enthält. Dabei kann der Wirkstoff mit einem Polymerisat enkapsuliert und anschließend in die Matrix eingelagert werden. Alternativ können die pulverförmigen Mischungspartikel von Matrixmaterial und Wirkstoff, d. h. die Einzelbestandteile des Depots, vor der Herstellung des Formkörpers beschichtet werden. Bei diesem Vorgehen ist eine Sinterung und damit eine Porenbildung nicht möglich, so daß das nach dieser Alternative hergestellte Calciumphosphat-Depot den Osteoblasten auch nicht die notwendige Tragfläche für die Anlagerung und Resorption bietet.

In der Zeitschrift Philips Technische Rundschau, Band 37, Nr. 9/10 1977/78 ist auf den Seiten 255 bis 257 die Eignung von Calciumphosphat für Implantationszwecke erläutert, und es sind verschiedene Möglichkeiten zur Erzeugung bestimmter Porositäten beim Sintern der Knochenimplantate beschrieben. Um neben den gewünschten großen Poren im Sinterkörper ausschließlich sehr kleine Poren zu erzeugen, ist in dieser Veröffentlichung ausgeführt, daß dem Calciumphosphat vor dem Sintern eine gewisse Menge Wasserstoffperoxid zugesetzt wird und daß die gewählte Menge sowie die Erwärmungsgeschwindigkeiten einen Einfluß auf die sich einstellende Makroporosität haben, während die Mikroporosität durch andere Parameter beeinflußt wird.

Durch die Druckschrift Clinical Orthopaedics and Related Research, Nr. 143, 1979, S. 266 bis 270 ist eine langdauernde antibakterielle Wirkung von Knochenzementen aus Polymethylmethacrylat homogenverteilten Silbersalzen bekannt.

Nach der DE-B-2 511 122 ist schließlich ein Vorprodukt für die Zubereitung von Knochenzement bekannt, das neben einer Gentamicinverbindung entweder pulverförmige Copolymerisate aus Methylmethacrylat und Methylacrylat oder monomeres Methylmethacrylat als Hauptbestandteil aufweist und das Gentamicin-Hydrochlorid und/oder Gentamicin-Hydrobromid oder ein Gemisch von Gentamicin-Sulfat mit Natriumchlorid, Kaliumchlorid, Natriumbromid und/oder Kaliumbromid enthält. Mit einem derartigen Vorprodukt soll eine Zubereitung von Knochenzement geschaffen werden, aus dem das Antibiotikum in höherer Konzentration freigesetzt wird.

Die Wirkstoffzugabe zu den Implantaten, wie beispielsweise deren Imprägnierung mit Breitbandmikrobiziden hat erfahrungsgemäß nur für einen gewissen Zeitraum Erfolg, nach dessen Ablauf die dann noch vorhandenen restlichen Wirkstoffmengen für eine therapeutische Wirkung zu klein sind. Restinfektionsherde oder spätere Sekundärinfektionen über den Blutkreislauf können dann an den Nahtstellen zwischen Implantat und Knochen zu Infektionen und Entzündungen führen, die meist den Erfolg der Implantation völlig in Frage stellen. Auch bei Verwendung von Kugelketten organischer Polymere, welche erhebliche Mengen Antibiotika enthalten, müssen diese nach einiger Zeit operativ entfernt werden, was mit einem nicht unerheblichen Risiko der Komplikation und Reinfektion verbunden ist.

Der Erfindung liegt die Aufgabe zugrunde, die Langzeitverträglichkeit von Knochenimplantaten zu verbessern und die Wahrscheinlichkeit von früher oder später auftretenden Komplikationen erheblich zu reduzieren.

Bei dem eingangs genannten Knochenimplantat besteht die erfindungsgemäße Lösung der Aufgabe darin, daß der Porositätsgrad größer ist als 50 % und das Knochenimplantat mit einem gewebeverträglichen, biologisch langsam abbaubaren Überzug bevorzugt aus einem Stoff der Gruppe der hochschmelzenden Fette, Polyglykolsäuren, Polyglygolsäure-Milchsäurecopolymere, alkylierten Zellulosederivate oder hochmolekularen Stärken überzogen ist sowie zusätzlich in die Kristallstruktur des Tricalciumphosphats eingebaute Silberionen enthält.

Bevorzugt wird hierbei, daß das Knochenimplantat aus einem bei ca. zwischen 950 und 1 400 °C gebrannten Gemisch aus 50 bis 90,5 Gewichtsteilen Tricalciumphosphat und 0,5 bis 50 Gewichtsteilen Silberphosphat besteht.

Erfindungsgemäß wird weiterhin ein Verfahren zum Herstellen eines Knochenimplantats aus Tricalciumphosphatkeramik mit mikroporöser und makroporöser Struktur vorgeschlagen, welches mit mindestens einem Breitbandmikrobizid imprägniert ist. Erfindungsgemäß ist bei diesem Verfahren vorgesehen, daß man Tricalciumphosphatpulver mit Wasser, gegebenenfalls Zusätzen zur Steuerung der Mikroporosität und Makroporosität und mit einem Silbersalz homogen vermischt und granuliert, dann bei Temperaturen zwischen 950 und 1 400 °C, vorzugsweise um 1 100 °C brennt, nach dem Abkühlen mit mindestens einem Mikrobizid tränkt und anschließend mit einem gewebeverträglichen, biologisch langsam abbaubaren Material überzieht, das bevorzugt einen Stoff aus der Gruppe der hochschmelzenden Fette, der Polyglykolsäuren, Polyglykolsäure-Milchsäurecopolymeren oder alkylierten Zellulosederivaten oder hochmolekularen Stärken enthält.

Bevorzugt wird das Tricalciumphosphatpulver zusammen mit den Zusätzen im Schlickerguß verformt und dann gebrannt. Hierbei ist es vorteilhaft, daß man ein Gemisch aus 50 bis 99,5 Gewichtsteilen Tricalciumphosphat und 0,5 bis 50 Gewichtsteilen Silberphosphat verwendet.

Ein weiteres Merkmal der Erfindung besteht schließlich in der Verwendung des erfindungsgemäßen Knochenimplantates als Überzug auf hochbelastbaren Endoprothesen. Bei diesen werden nach der Implantation ebenfalls Abwehrreaktionen des Körpers beobachtet, welche zum Teil erst lange nach der Implantation auftreten. Durch den erfindungsgemäßen Vorschlag kann auch hier eine deutliche Besserung erreicht werden.

Neben der angestrebten hohen Langzeitwirkung der erfindungsgemäßen Knochenimplantate ist deren Eignung besonders vorteilhaft als Prophylaxe der Osteomyelitis in osteomyelitischen Knochenhöhlen oder drittgradig offenen Frakturen.

Als Silbersalze kommen insbesondere Silberchlorid, Silbersulfat und Silberphosphat in Frage. In Fällen, wo die Farbe des Materials unerheblich ist, können prinzipiell auch Silberoxid und Silberpulver zur Anwendung kommen. In den Fällen, in denen das Material bei hohen Temperaturen gebrannt wird, kann das Silber selbstverständlich auch in Form von Silbernitrat oder Silberacetat zugefügt werden. Es ist anzunehmen, daß diese Silbersalze während des Brennvorganges sich zersetzen und die Silberkationen mehr oder weniger gleichmäßig in das Kristallgitter des Tricalciumphosphats mit eingebaut werden.

Insbesondere bei Verwendung von Silberphosphat findet dieser Einbau ohne große weitere Störungen des Kristallaufbaus statt. Trotzdem führt der Einbau von Silberkationen anstelle von Calciumkationen im Phosphatgitter zu einer derartigen Veränderung, daß die erhaltenen Produkte auch im porösen Zustand mechanisch wesentlich fester und widerstandsfähiger sind als silberfreie Tricalciumphosphate. An dieser Stelle sei erwähnt, daß Tricalciumphosphate im Sinne dieser Erfindung all die Produkte sind, bei denen das stöchiometrische Verhältnis zwischen Calciumoxid und $P_2O_5$ etwa 3 : 1 ist. Abweichungen nach unten und nach oben führen ebenfalls noch zu physiologisch verträglichen, mechanisch brauchbaren und vom Körper resorbierbaren Produkten.

In den nachfolgenden Beispielen sind einige Ausführungsformen des erfindungsgemäßen Materials sowie die Verfahren zu seiner Herstellung näher erläutert.

Beispiel 1

Handelsübliches Tricalciumphosphat (Best.-Nr. 2143 Merck) mit einer Korngröße von etwa 1 bis 2 μ wird durch Zusatz von Wasser oder 1 bis 3 %igen-wäßrigen Wasserstoffperoxidlösungen auf einem Tellergranuliergerät granuliert. Das verwendete Tellergranuliergerät hatte einen Tellerdurchmesser von 35 cm, einen Tellerrand von 7 cm, eine Neigung der Achse von 45°, eine Umdrehungsgeschwindigkeit von 45 Umdrehungen pro Minute und einen Materialdurchsatz von ca. 2 kg pro Stunde. Die Flüssigkeitsbeimengung beträgt ca. 30 Gew.-% des Pulvermaterials. Es werden etwa 500 g des Pulvers vorab durch einen gerührten Schütteltrichter mit etwa der doppelten Menge Flüssigkeit versehen, wobei die Flüssigkeit über eine Sprühpistole aufgegeben wird. Bereits nach einigen Minuten bildet sich das erste brauchbare Granulat. Es wird anschliessend kontinuierlich weiter Pulver und Flüssigkeit aufgegeben und kontinuierlich Granulat entnommen. Sofern ein kleineres Granulat gewünscht wird, können die Granula bei beliebiger Größe mit einem Löffel aus dem fließenden Verfahren herausgenommen werden. Bei Verwendung von Wasser mit Zimmertemperatur erhält man ein Granulat mit einer Porosität von etwa 60 %. Durch Zusatz von $H_2O_2$ wird die Porosität gesteigert. Die so erhaltenen Granula sind stabil genug, durch Sieben in Fraktionen verschiedener Größe aufgetrennt zu werden. Zu feinkörnige Granulate können erneut dem Granulierprozeß zugeführt werden. Zu große Korngrößen können nach entsprechender Zerkleinerung in das Granulierverfahren zurückgeführt werden.

Die fertigen Granulate werden anschließend gebrannt. Hierbei sind Temperaturen von 950 °C bis 1 400 °C geeignet. Die besten Ergebnisse werden erhalten, wenn die Granulate bei etwa 1 180 °C eine Stunde gebrannt werden. Man heizt 6 Stunden auf und Kühlt 24 Stunden ab. Solche Granulate sind mechanisch ausreichend fest, gut resorbierbar und geeignet, ausreichende Mengen eines Mikrobiozids oder Antibiotikums aufzunehmen.

Das so gewonnene Granulat wird im Wirbelstromverfahren mit einer Lösung von PVP-Jod besprüht und mit warmer Luft (ca. 50 °C) getrocknet. Bei einer Porosität von ca. 50 % kann ein Gehalt an Antibiotikumtrockensubstanz bis zu 30 % des Keramikausgangsgewichtes erreicht werden. Analoge Ergebnisse werden mit anderen Antibiotika, wie Phosphonycin, Flucloxacillin und dem Aminoglycoxid Certomycin® erzielt.

Ein mit einem Antibiotikum imprägniertes Granulat wird dragiert mit Polyglykolsäure gelöst in Hexafluorazeton oder Hexafluorisopropanol, wobei gegebenenfalls noch etwas Kresol zugegeben wird und auf mindestens 55 °C erwärmt wird. Man sprüht die Lösung mit auf 80-90 °C vorgewärmter Luft auf das Granulat. Die Polyglykolsäure fällt durch Abkühlung und Verdunstung des Lösungsmittels als Überzug aus.

Die so erhaltenen granulierten und imprägnierten Granulate sind für den Chirurgen leicht handhabbar. Als Antibiotikum wird jeweils dasjenige ausgesucht, welches aufgrund der vorher geprüften Resistenzlage optimal erscheint. Wegen der Resorbierbarkeit des Keramikmaterials muß dieses Material nicht später wieder entfernt werden. Es weist sofortige, mittelfristige und langfristige antiinfektiöse Eigenschaften auf. Es ist geeignet, auch bei zweitund drittgradig offenen Fraktionen die durchgeführte Osteosynthese durch Applikation dieses Granulats zu unterstützen und vor drohenden Infektionen und posttraumatischer Osteomyelitis zu schützen. Es ist kein Sekundäreingriff erforderlich und es ist nicht erforderlich, an einem anderen Ort körpereigenes Knochenmaterial zu entnehmen.

Beispiel 2

Ein mit einem Antibiotikum imprägniertes Granulat gemäß Beispiel 1 wird dragiert mit heißem, hochschmelzendem Fett (Dynasan 118 der Dynamit Nobel) mit einem Schmelzpunkt zwischen 69 und 71 °C. Dieses Fett wird auf 80 °C erwärmt und luftfrei mit einer Sprühpistole aus nächster Nähe auf das Granulat aufgesprüht, wobei sich eine Düse mit einem Durchlaß von 0,6 mm besonders bewährt hat. Es wird kontinuierlich aufgesprüht, damit immer frisch aufgesprühtes Fett auf der Oberfläche des Granulats liegt, da andernfalls ein gewisser Abrieb und Staubbildung auftreten können.

Das Material gemäß Beispiel 1 und Beispiel 2 hat bei operierten Hunden zu hervorragenden Ergebnissen geführt. Besonders bewährt hat sich auch das Einsetzen von Plomben, die aus einem Gemisch mehrerer dragierter Granulate hergestellt werden. Bedingt durch unterschiedliche Dicke des Überzuges wird offensichtlich der Wirkstoff noch gleichmäßiger freigesetzt, so daß über einen längeren Zeitraum ein ausreichender Wirkspiegel des Wirkstoffes aufrecht erhalten wird.

Beispiel 3

Handelsübliches Tricalciumphosphat mit einer Korngröße von etwa 1 bis 2 μ wird durch Zusatz von Wasser oder 1 bis 3 %igen wäßrigen Wasserstoffperoxidlösungen auf einem Tellergranuliergerät granuliert. Das verwendete Tellergranuliergerät hatte einen Tellerdurchmesser von 35 cm, einen Tellerrand von 7 cm, eine Neigung der Achse von 45°, eine Umdrehungsgeschwindigkeit von 45 Umdrehungen pro Minute und einen Materialdurchsatz von ca. 2 kg pro Stunde. Die Flüssigkeitsbeimengung beträgt ca. 30 Gew.-% des Pulvermaterials. Es werden etwa 500 g des Pulvers vorab durch einen gerührten Schütteltrichter mit etwa der doppelten Menge Flüssigkeit versehen, wobei die Flüssigkeit über eine Sprühpistole aufgegeben wird. Bereits nach einigen Minuten bildet sich das erste brauchbare Granulat. Es wird anschließend kontinuierlich weiter Pulver und Flüssigkeit aufgegeben und kontinuierlich Granulat entnommen. Sofern ein kleineres Granulat gewünscht wird, können die Granula bei beliebiger Größe mit einem Löffel aus dem fließenden Verfahren herausgenommen werden. Bei Verwendung von Wasser mit Zimmertemperatur erhält man ein Granulat mit einer Porosität von etwa 60 %. Durch Zusatz von $H_2O_2$ wird die Porosität gesteigert. Zur Herstellung von Granulaten mit einem Gehalt an Silbersalzen wird das entsprechende Silbersalz dem Ausgangsmaterial zugesetzt und homogen vermischt.

Die so erhaltenen Granula sind stabil genug, durch Sieben in Fraktionen verschiedener Größe aufgetrennt zu werden. Zu feinkörnige Granulate können erneut dem Granulierprozeß zugeführt werden. Zu große Korngrößen können nach entsprechender Zerkleinerung in das Granulierverfahren zurückgeführt werden. Der Zusatz verschiedener Mengen verschiedener Silbersalze hat praktisch keinen Einfluß auf das Granulierverfahren und die Eigenschaften der Granulate.

Die fertigen Granulate werden anschließend gebrannt. Hierbei sind Temperaturen von 950 bis 1 400 °C geeignet. Die besten Ergebnisse werden erhalten, wenn die Granulate bei etwa 1 100 °C eine Stunde gebrannt werden. Solche Granulate sind mechanisch ausreichend fest, gut resorbierbar und geeignet, ausreichende Mengen eines Mikrobiozids oder Antibiotikums aufzunehmen. Insbesondere bei Zusatz von Silberphosphat in Mengen von 10 bis 50 Gewichtsteilen Silberphosphat sind mechanisch wesentlich stabiler als silberfreie Granulate und Granulate mit geringem Silbergehalt. Die mechanische Stabilität hat keinen merklichen Einfluß auf die Resorbierbarkeit und Porosität.

Beispiel 4

Ein gemäß Beispiel 3 gewonnenes Granulat wird im Wirbelstromverfahren mit einer Lösung von Gentamycin besprüht und mit warmer Luft (ca. 50 °C) getrocknet. Bei einer Porosität von ca. 50 % kann ein Gehalt an Antibiotikumtrockensubstanz bis zu 30 % des Keramikausgangsgewichtes erreicht werden. Analoge Ergebnisse werden mit anderen Antibiotika erzielt.

Beispiel 5

Ein gemäß Beispiel 4 hergestelltes, mit einem Antibiotikum imprägniertes Granulat wird dragiert mit einer 5 %igen wäßrigen Lösung von Polyvinylpyrrolidon, Molekulargewicht 70 000. Analoge Ergebnisse werden erzielt mit einer 3 %igen wäßrigen Lösung von Dextran, Molekulargewicht größer als 100 000 und Polyglykolsäure gelöst in Hexafluorazeton oder Hexafluorisopropanol. Die so erhaltenen granulierten und imprägnierten Granulate sind für den Chirurgen leicht handhabbar. Als Antibiotikum wird jeweils dajenige ausgesucht, welches aufgrund der vorher geprüften Resistenzlage optimal erscheint. Wegen der Resorbierbarkeit des Keramikmaterials muß dieses Material nicht später wieder entfernt werden. Es weist sofortige, mittelfristige und langfristige antiinfektiöse Eigenschaften auf. Es ist geeignet, auch bei zweit- und drittgradig offenen Frakturen die durchgeführte Osteosynthese durch Applikation dieses Granulats zu unterstützen und vor drohenden Infektionen und posttraumatischer Osteomyelitis zu schützen. Es ist kein Sekundäreingriff erforderlich und es ist nicht erforderlich, an einem anderen Ort körpereigenes Knochenmaterial zu entnehmen.

**Patentansprüche**

1. Knochenimplantat aus Tricalciumphosphatkeramik mit mikroporöser und makroporöser Struktur, welches mit mindestens einem Breitbandmikrobizid imprägniert ist, dadurch gekennzeichnet, daß der Porositätsgrad größer ist als 50 % und das Knochenimplantat mit einem gewebeverträglichen, biologisch langsam abbaubaren Überzug bevorzugt aus einem Stoff der Gruppe der hochschmelzenden Fette, Polyglykolsäuren, Polyglykolsäure-Milchsäurecopolymere, alkylierten Zellulosederivate oder hochmolekularen Stärken überzogen ist sowie zusätzlich in die Kristallstruktur des Tricalciumphosphats eingebaute Silberionen enthält.

2. Knochenimplantat nach Anspruch 1, dadurch gekennzeichnet, daß es aus einem bei ca. zwischen 950 und 1 400 °C gebrannten Gemisch aus 50 bis 90,5 Gewichtsteilen Tricalciumphosphat und 0,5 bis 50 Gewichtsteilen Silberphosphat besteht.

3. Verfahren zum Herstellen eines Knochenimplantats aus Tricalciumphosphatkeramik mit mikroporöser und makroporöser Struktur, welches mit mindestens einem Breitbandmikrobizid imprägniert ist, dadurch gekennzeichnet, daß man Tricalciumphosphatpulver mit Wasser, gegebenenfalls Zusätzen zur Steuerung der Mikroporosität und Makroporosität und mit einem Silbersalz homogen vermischt und granuliert, dann bei Temperaturen zwischen 950 und 1 400 °C, vorzugsweise um 1 100 °C brennt, nach dem Abkühlen mit mindestens einem Mikrobizid tränkt und anschließend mit einem gewebeverträglichen, biologisch langsam abbaubaren Material überzieht, das bevorzugt einen Stoff der Gruppe der hochschmelzenden Fette, der Polyglykolsäuren, Polyglykolsäure-Milchsäurecopolymeren oder alkylierten Zellulosederivaten oder hochmolekularen Stärken enthält.

4. Verfahren nach Anspruch 3, dadurch gekennzeichnet, daß man das Tricalciumphosphatpulver zusammen mit den Zusätzen im Schlickerguß verformt und dann brennt.

5. Verfahren nach Anspruch 4, dadurch gekennzeichnet, daß man ein Gemisch aus 50 bis 99,5 Gewichtsteilen Tricalciumphosphat und 0,5 bis 50 Gewichtsteilen Silberphosphat verwendet.

6. Verwendung eines Knochenimplantats nach einem der Ansprüche 1 und 2 als Überzug auf hochbelastbaren Endoprothesen.

**Claims**

1. Bond implant of tricalcium phosphate ceramics having a microporous and macroporous structure, which is impregnated with at least one broad-band microbicide, characterised in that the degree of porosity is greater than 50 % and the bone implant is covered with a coating which is compatible with the material and which biologically is slowly degradable, preferably consisting of a substance in the group of high-melting fats, polyglycolic acids, copolymers of polyglycolic acid and lactic acid, alkylated cellulose derivatives or high molecular weight starches and additionally contains silver ions incorporated into the crystal structure of the tricalcium phosphate.

2. Bone implant according to claim 1, characterised in that it consists of a mixture of 50 to 90.5 parts by weight of tricalcium phosphate and 0.5 to 50 parts by weight of silver phosphate, which mixture is fired at between about 950 and 1 400 °C.

3. Process for the production of a bone implant of tricalcium phosphate ceramics having a microporous or macroporous structure, which is impregnated with at least one broad-band microbicide, characterised in that tricalcium phosphate powder is homogeneously mixed with water, possibly additives for controlling the microporosity and macroporosity and with a silver salt and granulated, is then fired at temperatures between 950 and 1 400 °C, advantageously in the region of 1 100 °C, is impregnated after the cooling with at least one microbicide and thereafter is coated with a tissuecompatible material which biologically can be slowly degraded and which preferably contains a substance included in the group of the high-melting fats, polyglycolic acids, copolymers of polyglycolic acid and lactic acid, or alkylated cellulose derivatives or starches of high molecular weight.

4. Process according to claim 3, characterised in that the tricalcium phosphate powder is shaped together with the additives in the slip casting and is then fired. .

5. Process according to claim 4, characterized in that a mixture of 50 to 99.5 parts by weight of tricalcium phosphate and 0.5 to 50 parts by weight of silver phosphate is used.

6. Use of a bone implant according to one of the claims 1 and 2 as a covering on endoprotheses which can withstand a high load.

**Revendications**

1. Implant osseux à base de céramique de phosphate tricalcique à structure microporeuse et macroporeuse, qui est imprégnée avec au moins un microbicide à large spectre, caractérisé en ce que le degré de porosité est supérieur à 50 % et que l'implant osseux est revêtu avec un revêtement compatible avec les tissus et se dégradant lentement de façon biologique, de préférence en un produit du groupe des corps gras à point de fusion élevé, des acides polyglycoliques, des copolymères d'acide polyglycolique et d'acide lactique, des dérivés cellulosiques alcoylés ou des amidons de masse moléculaire élevée et contenant de plus des ions argent insérés dans la structure cristalline du phosphate tricalcique.

2. Implant osseux selon la revendication 1, caractérisé en ce qu'il se compose d'un mélange, cuit entre 950 et 1 400 °C environ, de 50 à 90,5 parties en poids de phosphate tricalcique et de 0,5 à 50 parties en poids de phosphate d'argent.

3. Procédé pour fabriquer un implant osseux à base de céramique de phosphate tricalcique à structure microporeuse et macroporeuse, qui est imprégné avec au moins un microbicide à large spectre, caractérisé en ce qu'on mélange de façon homogène et on met sous forme de granules de la poudre de phosphate tricalcique avec de l'eau, le cas échéant avec des additifs pour commander la microporosité et la macroporosité et avec un sel d'argent, on la cuit ensuite à des températures comprises entre 950 et 1 400 °C, de préférence voisines de 1 100 °C, on l'imprègne après refroidissement avec au moins un microbicide et on la revêt ensuite avec un matériau compatible avec les tissus et se dégradant lentement de façon biologique, ce matériau contenant de préférence un produit du groupe des corps gras à point de fusion élevé, des acides polyglycoliques, des copolymères d'acide polyglycolique et d'acide lactique ou des dérivés cellulosiques alcoylés ou des amidons de masse moléculaire élevée.

4. Procédé selon la revendication 3, caractérisé en ce qu'on façonne la poudre de phosphate tricalcique conjointement avec les additifs par coulée en barbotine et on la cuit ensuite.

5. Procédé selon la revendication 4, caractérisé en ce qu'on utilise un mélange de 50 à 99,5 parties en poids de phosphate tricalcique et 0,5 à 50 parties en poids de phosphate d'argent.

6. Utilisation d'un implant osseux selon l'une des revendications 1 et 2 en tant que revêtement sur des prothèses d'extrémité fortement chargées.